# NEUE EUROPÄISCHE PATENTSCHRIFT

(11) **EP 1 534 234 B2**
(45) Veröffentlichungstag und Bekanntmachung des Hinweises auf die Entscheidung über den Einspruch: **24.07.2013**
(45) Hinweis auf die Patenterteilung: 23.12.2009
(21) Anmeldenummer: 03793760.4
(22) Anmeldetag: 28.08.2003
(51) Int. Cl.: A61Q 19/00, A61K 8/64, A61K 8/97, A61K 8/92

(54) **ZUSAMMENSETZUNG UND DEREN VERWENDUNG ALS PHARMAZEUTISCHE ODER KOSMETISCHE FORMULIERUNG ZUR ÄUSSERLICHEN ANWENDUNG**
COMPOSITION AND USE THEREOF AS A PHARMACEUTICAL OR COSMETIC FORMULATION FOR EXTERNAL APPLICATION
COMPOSITION ET SON UTILISATION COMME FORMULATION PHARMACEUTIQUE OU COSMETIQUE POUR UNE APPLICATION EXTERNE

(30) Priorität: 05.09.2002 DE 10240989
(43) Veröffentlichungstag der Anmeldung: 01.06.2005
(73) Patentinhaber: Klewinghaus, Maria, 40765 Langenfeld (DE); Klewinghaus, Uwe, 40765 Langenfeld (DE)
(72) Erfinder: Klewinghaus, Maria, 40765 Langenfeld (DE)
(74) Vertreter: Gille Hrabal
(86) Internationale Anmeldenummer: PCT/EP2003/009506
(87) Internationale Veröffentlichungsnummer: WO 2004/022026

(56) Entgegenhaltungen:
- EP-A2- 0 334 507
- WO-A-03/094881
- WO-A1-97/23198
- WO-A1-99/46273
- DE-A- 3 431 845
- DE-C- 4 205 783
- DE-C- 4 302 132
- MERCK & CO. INC.: 'The Merck Index, Thirteen Edition' MERCK INDEX 2001, WHITEHOUSE STATION, NJ, Seite 987

## Beschreibung

Die Erfindung betrifft eine kosmetische Zusammensetzung zur äußerlichen kosmetischen Anwendung bei Menschen oder Tieren, ein Verfahren zu Ihrer Herstellung sowie die nicht-therapeutisch Verwendung der Zusammensetzung zur Behandlung von trockener Haut und/oder zur regenerierenden Behandlung der Haut.

Glycerin ist ein bekanntes Humektant in Hautpflegeprodukten. Humektantien (Feuchthaltemittel) sind Substanzen, deren Aufgabe es ist, Wasser nach dessen Diffusion in die Haut dort zu binden. Glycerin verleiht den Pflegeprodukten außerdem eine angenehme Konsistenz. Es ist eine farb- und geruchslose, ölige, süß schmeckende ungiftige Flüssigkeit: In höheren Konzentrationen reizt es jedoch die Schleimhäute und kann unter Umständen stark nachteilig wirken, wenn es wegen geringer Luftfeuchtigkeit der Haut Wasser entzieht und diese dadurch austrocknet und rissig wird. Deshalb und wegen seiner Klebrigkeit auf der Haut wird es durch andere Stoffe, wie z.B. Propylenglykol ersetzt (s. z.B. Aggy und Frank Burczyk, Kosmetiklexikon, Ehrenwirth, München 1993). Kosmetische Cremes mit höheren Glycerinanteilen sind daher im allgemeinen weniger bevorzugt.

Ungesättigte Fettsäuren gehören, sofern sie mehrfach ungesättigt sind, zu den als essentiell bezeichneten Fettsäuren (Llnol-, Linolen-, Arachidonsäure etc.). Bei ihrer Anwendung in Formulierungen für die äußere Anwendung muß beachtet werden, dass die ungesättigten Fettsäuren und auch deren Glyceride bzw. Ester oxidationsempfindlich sind und auf der Humanhaut schon nach kurzer Zeit hautlrrltierende Spaltprodukte bilden können. Es wird daher empfohlen, die ungesättigten Fettsäuren stets unter gleichzeitigem Zusatz von Antioxidantien zu verarbeiten (Fiedler - Lexikon der Hilfsstoffe, Editio Cantor Verlag, Aulendorf, 2002). Aus diesem Grund ist die Verwendung von Formulierungen für die äußere Anwendung mit einem hohen Gehalt an ungesättigten Fettsäuren bzw. Glyceriden bzw. Estern davon an sich nicht bevorzugt. Weiterhin kann es beim Einsatz der Antioxidantien wie eines Gemisches von Ethoxyquin und Butylhydroxytoluol bei empfindlichen Hauttypen zu allergischen Reaktionen kommen, was ebenfalls nicht erwünscht ist.

Zu den ungesättigten Fettsäuren gehört insbesondere auch die Linolensäure, eine dreifach ungesättigte essentielle Fettsäure, die als Glycerinester in vielen Pflanzenölen vorkommt. In der Natur kommen zwei Stellungsisomere der Linolensäure vor, die α-Linolensäure ((*all*-*Z*)-9,12,15-Octadecatriensäure) und die γ-Linolensäure (*(all*-*Z*)-6,9,12-Octadecatriensäure). Während die α-Linolensäure als Glycerinester in vielen Pflanzenölen vorkommt, kommt die γ-Linolensäure nur In wenigen Pflanzenölen als Glycerinester vor, z.B. im Samenöl von Nachtkerze (*Oenothera blennis*, ca. 10 %), schwarzer Johannisbeere (*Ribes nigrum*, ca, 15 %), Borretsch (*Borago officinalis*, 20-25 %) und anderen Rauhblattgewächsen sowie In Algenpilzen (Phycomycetes) (s, z.B. Römpp Lexikon, Naturstoffe, Thieme, 1997). Sowohl Nachtkerzenöl als auch Borretschöl tendieren wie die übrigen ungesättigten Fettsäuren dazu, sich in Formulierungen für die äußerliche Anwendung instabil zu verhalten und dabei auch einen speziellen, unangenehmen Geruch zu entwickeln (S. Borelli et al.; H+G, Band 69, Heft 8, 1994: Externe Theraphie mit γ-Linolensäure - Ergebnis einer Doppelblindstudie). Daher ist ihre Anwendung in Formulierungen für die äußerliche Anwendung ebenfalls nicht bevorzugt.

Aus der DE 3431845 ist ein Verfahren zur Herstellung kosmetischer und/oder pharmazeutischer Cremes, mit Extrakten aus spurenelementenreichen Kräutern, für die Regeneration und Stabilisierung der menschlichen Haut bekannt, das dadurch gekennzeichnet ist, dass man eine Mischung aus 5-70% aus pflanzlichen oder tierischen Fetten gewonnenen stearinarmen oder stearinfreien Öles, 5-20% für pharmazeutische oder kosmetische Zwecke verwendbares Glyzerin (in einer Konzentration bis 95%) und Wasser emulgiert, anschliessend homogenisiert und unter Zugabe von Auszügen aus spurenelementreichen Kräutern zu einer pastösen Masse abkühlt. Das Glycerin wird dabei als Wirkstoffträger sowie als Konservierungsstoff für die aufgeschlossenen Spurenelemente benutzt. Die Natur der stearinarmen oder stearinfreien Fette oder Öle und ihr Stearin-Gehalt wird nicht näher beschrieben. Auch werden keine spezifischen Beispiele von formulierungen offenbart.

Die DE 692 13 930 (entsprechend EP 0 575 461) betrifft eine Fettzusammensetzung für kosmetische oder pharmazeutische Emulsionsprodukte, die eine bestimmte Mischung von Mono-, Di- und Triglyceriden aufweist. In den konkreten Beispielen (vgl, Seite 9, Tabelle) wird lediglich 3 Gew.-% Glycerin verwendet. Eine Menge von 5 bis 20 Gew.-% Glycerin (Seite 5, Zeile 1) wird lediglich im Zusammenhang mit der Herstellung des sogenannten Glyceridsystems A erwähnt.

Die EP 0 689 456 B1 offenbart eine Hautpflegezusammensetzung, die eine primäre Ölphase und ein Emulgiermittel basierend auf einer Mischung aus Sorbitanfettsäureester und Saccharosefettsäureester enthält. Im Patentanspruch 9 wird auch offenbart, ein Feuchthaltemittel (Humectant), ausgewählt aus verschiedenen Verbindungen, darunter auch Glycerin, in einer Menge von 0,1 bis 20 Gew.-% zu verwenden. In den konkreten Beispielen 1 bis V auf Seite 6 (Tabelle) wird Glycerin tatsächlich jedoch nur in Mengen von 2 bis 3 Gew.-% verwendet. Dies bestätigt erneut, dass Hautpflegezusammensetzungen mit mehr als 5 Gew.-% Glycerin unüblich sind. Weiterhin heißt es auf Seite 5, Zeile 1, dass bevorzugt lediglich 2 bis 5 Gew.-% der Zusammensetzung durch das Feuchthaltemittel (Humectant) gebildet werden. Auch werden In den Beispielen nur gesättigte Fettsäuren (Stearinsäure) verwendet. Hinweise auf die kombinierte Verwendung eines hohen Glyceringehalts mit ungesättigten pflanzlichen Ölen finden sich in dieser Druckschrift nicht.

In der DE 694 17 726 (entsprechend EP 0682006 B1) ist ein Verfahren beschrieben, mit dem man ein Konzentrat von Ethylestern mehrfach ungesättigter Fettsäuren, darunter auch von Gamma-Linolensäure, erhält. Konkrete kosmetische oder pharmazeutische Zusammensetzungen sind zwar erwähnt, im einzelnen jedoch nicht beschrieben. Auch die Anwendung von Glycerin in einer Menge von 5 bis 25 Gew.-% ist diesem Dokument an keiner Stelle zu entnehmen.

In der US-6 103 246 wird eine sehr spezielle Zusammensetzung für die äußere Anwendung auf der menschlichen Haut beschrieben, die neben dem tierischen Öl "Emu-Öl" 2 bis 9,5 Gew.-% Glycerin enthält. Die Anwendung tierischer Fette oder Öle begegnet jedoch häufig ethischen Bedenken. Zutreffend wird auch in dieser Anmeldung erwähnt, dass tierische Öle im allgemeinen in traditionellen kosmetischen Formulierungen nicht verwendet werden (Spalte 2, Zellen 5 bis 7). Hinweise auf die kombinierte Verwendung von bestimmten pflanzlichen Ölen und hohen Glycerinantellen finden sich in dieser Druckschrift nicht.

Die vorliegenden Erfinder fanden nun, das eine Zusammensetzung, die gleichzeitig einen relativ hohen Gehalt an Glycerin sowie einen Gehalt an ungesättigten Fettsäuren bzw. deren Glyceride aufweist, die oben geschilderten Nachteile, die sonst mit der Anwendung eines hohen Glyceringehalts oder eines Gehalts ungesättigter Fettsäuren bzw. deren Glyceride allein verbunden sind, überraschend nicht auftreten. Insbesondere wird die mit der Anwendung von Glycerin In hohen Konzentration In kosmetischen Formulierungen verbundene nachteilige Wirkung, wie insbesondere das Austrocknen der Haut, verhindert. Gleichzeitig tritt die mit der Anwendung der ungesättigten Fettsäuren verbundene Bildung hautirritierender Spaltprodukte, auch wenn keine Konservierungsstoffe, wie Antioxidationsmittel verwendet werden, nicht merklich auf, so dass auf deren Einsatz vollständig verzichtet werden kann. Es werden kosmetische Zusammensetzungen für die äußere Anwendung erhalten, die überraschend eine sehr hohe Hautvertröglichkeit besitzen, und zur regenerierenden Behandlung insbesondere von trockener Haut geeignet sind. Dabei führt insbesondere der Zusatz von γ-Linolensäure-haltigen pflanzlichen Ölen zu sehr guten Hautwirkungen. Es wird somit eine kosmetische Formulierung für die äußerliche kosmetische Anwendung bereitgestellt, die eine stark regenerierende und feuchtigkeitserhaltende Wirkung auf die Haut besitzt und insbesondere eine vorhandene Trockenheit der Haut beseitigt. Dies war bei einer Creme mit derartig hohem Glyceringehalt nicht zu erwarten. Die kosmetische Formulierung ist daher besonders geeignet zur nicht-therapeutischen Behandlung trockener Haut. Weiterhin war es, wie gesagt überraschend, dass die Stabilltätsprobieme, die Insbesondere mit der Verwendung von γ-Linolensäure-haltigen Ölen verbunden sind, in der erfindungsgemäßen Zusammensetzung gelöst werden, so dass auf den Einsatz von Konservierungsstoffen verzichtet werden kann. Die erfindungsgemäße Zusammensetzung ist daher besonders verträglich und eignet sich hervorragend sowohl als Basisfettcreme als auch als Trägercreme für beliebige weitere kosmetische oder pharmazeutische Wirkstoffe. Die erfindungsgemäße Zusammensetzung verwendet weiterhin Fettsubstanzen auf pflanzlicher Basis.

Die vorliegende Erfindung stellt somit eine neue kosmetische Zusammensetzung, ein Verfahren zu ihrer Herstellung sowie ihre nicht-therapeutische Verwendung als kosmetische Formulierungen zur äußerlichen Anwendung bereit.

Die Erfindung betrifft eine Kosmetische Zusammensetzung zur äußerlichen kosmetischen Anwendung bei Menschen oder Tieren, die besteht aus:
- 12 bis 20 Gew.-% Glycerin, bezogen auf die Gesamtmenge der Zusammensetzung,
- mindestens 20-55 Gew.-% bezogen auf die Gesamtmenge der Zusammensetzung einer Fettsubstanz pflanzlicher Herkunft, enthaltend mindestens eine mehrfach ungesättigten Fettsäure in freier oder chemisch gebundener Form,
- Wasser,
- gegebenenfalls einen oder mehrere Emulgatoren sowie
- gegebenenfalls einen oder mehrere kosmetische und/oder pharmazeutische Wirkstoffe, sowie gegebenfalls einen oder mehrere übliche Zusatzstoffe, jeweils wie in Anspruch 1 definiert,
   wobei die Zusammensetzung einen y-Linolensäure-Gehalt von mindestens 0,005 Gew.-%, bezogen auf die Gesammtmenge der Zusammensetzung aufweis, und wobei die Zusammensetzung keine Wachse, keine Paraffine und keine Silikonöle enthält.

Bei dem erfindungsgemäß eingesetzten Glycerin handelt es sich um für kosmetische oder pharmazeutische Zwecke geeignetes Glycerin, Bevorzugt wird erfindungsgemäß Glycerin aus pflanzlicher Herkunft verwendet. Die Menge des Glycerins in der erfindungsgemäßen Zusammensetzung beträgt 12 bis 20 Gew.-% bezogen auf die Gesamtmenge der Formulierung. Ein Gehalt von weniger als 5 Gew.-% führt zu einer Verringerung der feuchtigkeitsspendenden Wirkung. Mit Gehalten von mehr als 25 Gew.-% Glycerin lässt sich keine Steigerung der günstigen Wirkungen des Glycerins mehr erzielen und die negativen Wirkungen des Glycerins überwiegen. Bevorzugt liegt der Glyceringehalt der erfindungsgemäßen Zusammensetzung bei mehr als 5 Gew.-% bis 25 Gew.-%, noch bevorzugter bei 12 bis 20 Gew.-%.

Der Begriff des "Glyceringehalts", wie er in der vorliegenden Erfindung verwendet wird, bezeichnet den Gehalt an freiem Glycerin in der Zusammensetzung, schließt also den Gehalt an chemisch gebundenem Glycerin, wie den Glyceringehalt der verwendeten Fett und/oder Öle nicht mit ein.

Die erfindungsgemäße Zusammensetzung enthält mindestens eine Fettsubstanz pflanzlicher Herkunft, enthaltend mindestens eine mehrfach ungesättigten Fettsäure In freier oder chemisch gebundener Form. Eine Fettsubstanz pflanzlicher Herkunft bedeute, dass sie aus Pflanzen oder Pflanzentellen gewonnen wurde, wobei eine anschließende chemische oder physikalische Modifizierung nicht ausgeschlossen ist. Es kann sich um Mischungen von Fettsubstanzen, die keine mehrfach ungesättigten Fettsäuren enthalten, und Fettsubstanzen, die mehrfach ungesättigten Fettsäuren enthalten, handeln. Die Fettsubstanzen pflanzlicher Herkunft können bevorzugt aus für kosmetische oder pharmazeutische Formulierungen gebräuchlichen Fetten, Ölen und Wachsen und Mischungen davon ausgewählt werden. Die Fettsubstanzen sind pflanzlicher Herkunft, insbesondere pflanzliche Öle, die im wesentlichen aus gemischten Glycerinestern höherer Fettsäuren mit gerader Anzahl von Kohlenstoffatomen bestehen, wie Aprikosenkernöl, Avocadoöl, Baumwollöl, Borretschöl, Distelöl, Erdnussöl, gehärtetes Erdnussöl, Getreidekeimöl, Hanföl, Haselnussöl, Kürbiskernöl, Kokosöl, Leinsamenöl, Lorbeeröl, Mohnöl, Macadamiaöl, Maisöl, Mandelöl, Nachtkerzenöl, Olivenöl, hydriertes Palmöl, Palmöl, Pistazienkernöl, Rapsöl, Rizinusöl, Sanddornöl, Sesamöl, Sojaöl, Sonnenblumenkernöl, Traubenkernöl, Walinussöl, Weizenkelmöl, Wildrosenöl, Kokosfett, Palmfett, Palmkernfett oder Rüböl, Wachse, bei denen es sich im engeren Sinne um eine Sammelbezeichnung für Ester langkettiger Fettsäuren mit langkettigen Alkoholen handelt, sind ausgeschlossen. Pflanzliche Wachse schließen beispielsweise Carnaubawachs und Japan-Wachs ein. Wachse sind jedoch nicht in der erfindungsgemäßen Zusammensetzung enthalten. Auch Paraffine und Silikonöle sind nicht in der erfindungsgemäßen Zusammensetzung enthalten. Fettsubstanzen können ferner Fettsäuren selbst und deren Derivate, insbesondere Ester (Glycerinester etc.) einschließen. Die verwendete Fettsubstanz enthält mindestens eine mehrfach ungesättigte Fettsäure in freier oder In chemisch gebundener Form. Bevorzugt ist die mehrfach ungesättigte Fettsäure In chemisch gebundener Form, bevorzugter als Triglycerid von Fettsäure(n), ganz besonders bevorzugt in der Form pflanzlicher Fette und/oder Öle enthalten. Bevorzugt enthält die Zusammensetzung der Erfindung eine Mehrzahl bzw. eine Mischung von mindestens zwei pflanzlichen Fetten und/oder Ölen mit einem Gehalt an mehrfach ungesättigten Fettsäuren.

Bei den bevorzugt verwendeten pflanzlichen Fetten und/oder Ölen, die die mehrfach ungesättigten Fettsäure(n) enthalten, handelt es sich um solche, die einen sogenannten P/S-Quotienten von mindestens etwa 0,5 aufweisen. Werden Mischungen von Fetten und/oder Ölen verwendet, weist mindestens eines der verwendeten Fett und/oder Öl einen P/S-Quotienten von mindestens etwa 1, bevorzugter mindestens etwa 2 auf. Noch bevorzugter weist mindestens eines der verwendeten Fette und/oder Öle einen P/S-Quotienten von mindestens etwa 3, gelegentlich mindestens etwa 5 auf.

Bei dem sogenannten P/S-Quotlenten handelt es sich um das Gewichtsverhältnis des Gehalts der mehrfach ungesättigten Fettsäuren zu dem Gehalt der gesättigten Fettsäuren, die in einem Fett und/oder Öl enthalten sind. Der P/S-Quotient für ein gegebenes Fett und/oder Öl wird bestimmt durch die Bestimmung des prozentualen Gehalts der mehrfach ungesättigten Fettsäuren, bezogen auf die Gesamtmenge der Fettsäuren und die Bestimmung des prozentualen Gehalts der gesättigten Fettsäuren, bezogen auf die Gesamtmenge der Fettsäuren, und Bildung des Quotienten:
"Prozentualer Gehalt der mehrfach ungesättigten Fettsäuren, bezogen auf die Gesamtmenge der Fettsäuren / Prozent-ualer Gehalt der mehrfach ungesättigten Fettsäuren, bezogen auf die Gesamtmenge der Fettsäuren".

Die Bestimmung des Fettsäuregehalts von Fetten und/oder Ölen und die anschließende Berechnung des resultierenden P/S-Quotienten erfolgt dabei wie in P. Schweiger, S. Kerschmann in "Untersuchungen über die Fettsäure- und Tocopherolgehalte von Pflanzenölen", Abschlußbericht über das Arbeitsprojekt ,Pflanzenöle' der Landesanstalt für Pflanzenbau, Forchheim, Kapitel 3.2 beschrieben (s.a. "Rohfettgehalt und Fettsäurezusammensetzung verschiedener Pflanzenöle", Abschlußbericht über das Arbeitsprojekt 'Pflanzenöle' der Landesanstalt für Pflanzenbau, Forchheim). D.h., die Bestimmung der Fettsäuremuster erfolgt mittels Gaschromatographie, wobei die Fettsäuretriglyceride aufgrund ihrer Schwerflüchtigkeit zu Methylestern mit Trimethylsulfoniumhydroxid (TSH) umgeestert werden. Dabei werden 50 mg Öl In einen 5 ml Schilffkolben eingewogen und mit 5 ml n-Heptan versetzt. Dazu werden 0,5 ml TSH-Lösung gegeben und gut durchgeschüttelt und über Nacht in den Kühlschrank gestellt. Überschüssiges Methanol setzt sich dabei am Boden des Kölbchens ab. Von der klaren n-Heptanphase wird 1 ml in den Gaschromatograph eingespritzt. Die Herstellung des Methyllerungsreagenz und der Standardlösung erfolgt wie folgt: Eine 200 mm x 20 mm Glaspürette wird mit einem Glaswollpfropfen versehen und mit 35 ml Ionenaustauscher Amberlite IRA-420, der mit Wasser aufgeschlämmt ist, gefüllt. Danach wird der lonenaustauscher mit 150 ml 4% NaOH-Lösung sowie 150 ml Wasser gewaschen, bis der Ablauf neutral reagiert. Zuletzt erfolgt noch eine Spülung mit 150 ml Methanol, 4,1 g (0,02 mol) Trimethylsulfoniumlodid werden unter Erwärmen auf 50° C in 60 ml Methanol gelöst und in 5 bis 10 ml Portionen über den lonenaustauscher gegeben. Zum Schluß wird mit 60 ml Methanol nachgewaschen. Die TSH-Lösung wird im Kühlschrank aufbewahrt.

### GC-Parameter

Die GC-Analysen der Fettsäuren werden unter folgenden Bedingungen durchgeführt:
GC: Sichromat; Detektor: FID; Säule: DB-WAX 60 m x 0.25 mm Temperaturprogramm: Ofentemperatur 150°C Zeit: 5 min; Aufheizgeschwindigkeit: 10°C/min
Ofentemperatur: 225°C; Zeit: 12 min; Aufheizgeschwindigkeit: 3°C/min Ofentemperatur: 250°C; Zeit : 20 min (bei Amaranth und Quinoa 30 min)
Injektortemperatur: 250°C; Detektortemperatur: 300°C
Die GC/MS-Untersuchungen werden durchgeführt mit:
GC: HP 5890 Series II; MS: HP 5972 Mass Selectiv Detektor; Säule: DB 17 30 m x.25 mm

Zur Ermittlung der Retentionszeiten der Fettsäuremethylester werden 3 verschiedene Standardlösungen der Firma ROTH (Rotichrom FO2, Rotichrom FO3 und Rotichrom ME29) verwendet. Die Messungen werden In regelmäßigen Abständen wiederholt. Die ermittelten Retentionszeiten nehmen Innerhalb von 6 Monaten erfahrungsgemäß ab. Die Retentionszeit für γ-Linolensäure, Eicosadiensäure, Rizinolsäure, Laurinsäure, Caprylsäure und Caprinsäure sowie Squalen wird aus Literaturwerten bestimmt und mit GC/MS nachgeprüft.

Die folgende Tabelle zeigt die häufigsten, natürlich vorkommenden Fettsäuren (nach P. Schweiger, S. Kerschmann in "Untersuchungen über die Fettsäure- und Tocopherolgehalte von Pflanzenölen", ibid.):

| Fettsäuren | Chemische Kurzform | Trivialbezeichnung | Chemische Bezeichnung |
|---|---|---|---|
| gesättigt: | C4:0 | Buttersäure | Butansäure |
| | C6:0 | Capronsäure | Hexansäure |
| | C8:0 | Caprylsäure | Oktansäure |
| | C10:0 | Caprinsäure | Decansäure |
| | C12:0 | Laurinsäure | Dodecansäure |
| | C14:0 | Myristinsäure | Tetradecansäure |
| | C16:0 | Palmitinsäure | Hexadecansäure |
| | C18:0 | Stearinsäure | Octadecansäure |
| | C20:0 | Arachinsäure | Eicosansäure |
| | C22:0 | Behensäure | Docosansäure |
| | C24:0 | Lignocerinsäure | Tetracosansäure |
| | C26:0 | Cerotinsäure | Hexacosansäure |
| einfach ungesättigt | C14:1 | Myristoleinsäure | 9:10 Tetradecensäure |
| | C16:1 | Palmitoleinsäure | 9:10 Hexadecensäure |
| | C18:1 | Ölsäure | 9:10 Oktadecensäure |
| | C18:1 | Vaccensäure | 11:12 Oktadecensäure |
| | C18:1 | Petroselinsäure | 6:7 Oktadecensäure |
| | C20:1 | Gadoleinsäure | 9:10 Eicosensäure |
| | C20:1 | | 11:12 Eicosensäure |
| | C22:1 | Erucasäure | 12:14 Docosensäure |
| | C24:1 | Nervonsäure | 15:16 Tetracosensäure |
| zweifach ungesättigt | C18:2 | Linolsäure | 9:10 12:13 Octadecadiensäure |
| | C20:2 | | 8:9 11:12 Eicosadiensäure |
| dreifach ungesättigt | C18:3 | α-Linolensäure | 9,12,15 Octadecatriensäure |
| | C18:3 | γ-Linolensäure | 6,9,12 Octadecatriensäure |
| | C20:3 | | 8,11,14 Eicosatriensäure |
| | C20:3 | | 11,14,17 Eicosatriensäure |
| vierfach ungesättigt | C18:4 | Stearidonsäure | 6,9,12,15 Octadecatetraensäure |
| | C20:4 | Arachidonsäure | 5,8,11,14 Eicosatetraensäure |
| | C22:4 | | 7,10,13,16 Docosatetraensäure |
| fünffach ungesättigt | C20:5 | Timnodonsäure | 5,8,11,14,17 Eicosapentaensäure (EPA) |
| | C22:5 | Clupanodonsäure | 4,8,12,15,19 Docosapentaensäure |
| sechsfach ungesättigt | C22:6 | Cervonsäure | 4,7,10,13,16,19 Docosahexaensäure (DHA) |

Bis auf Buttersäure, Capronsäure, EPA und DHA lassen sich alle gelisteten Fettsäuren in Pflanzenölen und -fetten finden. Fettsäuren mit mehr als drei Doppelbindungen kommen jedoch nur in sehr geringen Mengen vor, wobei Stearidonsäure die in Hanföl in einer Konzentration von ca. 1 % vorkommt, eine Ausnahme bildet. In Pflanzenölen häufig vorkommende, mehrfach ungesättigte Fettsäuren sind insbesondere Linolsäure und Linolensäure.

Die P/S-Quotienten können natürlich abhängig von der Herkunft der pflanzlichen Fette und/oder Öle in gewissen Bereichen schwanken. Zu den Ölen mit einem sehr hohem Anteil an mehrfach ungesättigten Fettsäuren (über 65 %), die einen P/S-Quotienten von > 5,5 aufweisen, gehören beispielweise Distelöl, Sonnenblumenöl (normal), Hanföl, Wildrosenöl, Tabaköl, Königskerzenöl, Mohnöl, Leinsamenöl, Nachtkerzenöl und Walnussöl. Die erfindungsgemäße Zusammensetzung enthält bevorzugt mindestens eines dieser Öle.

Zu den Ölen mit einem hohen Anteil an mehrfach ungesättigten Fettsäuren (über 50 bis 65 %), die einen P/S-Quotienten von etwa 3,8 bis 5,5 aufweisen, gehören beispielsweise Traubenkernöl, Schwarzkümmelöl, Borretschöl, Kürbiskernöl, Sojaöl und Weizenkelmöl.

Zu den Ölen mit einem P/S-Quotienten von etwa 2.2 bis 3.8 gehören beispielsweise Rapsöl, Haseinussöl, Erdnußöl, Aprikosenkernöl, Mandelöl, Sonnenblumenöl (high-oleic) und Pistazienöl.

Öle mit einem P/S-Quotienten von < 1 sind beispielweise Olivenöl, Macadamiaöl, Avocadoöl und Sanddornöl.

Die Zusammensetzung enthält mindestens 20 bis 55 Gew.-% der Fettsubstanz planzlicher Herkunft.

Die erfindungsgemäß verwendete pflanzliche Fettsubstanz umfasst bevorzugt mindestens ein Fett und/oder Öl, das mindestens eine mehrfach ungesättigte Fettsäuren enthält. Bevorzugt besteht die Fettsubstanz zu mindestens 20, bevorzugter zu mindestens 40, besonders bevorzugt mindestens 60, ganz bevorzugt zu mindestens 80 Gew.-% Fetten und/oder Ölen mit einem Gehalt an mehrfach ungesättigten Fettsäuren, bezogen auf die Gesamtmenge der Fettsubstanzen. In der am meisten bevorzugten Ausführungsform besteht die erfindungsgemäß verwendete Fettsubstanz zu 100 Gew.-% aus Fetten und/oder Ölen, insbesondere pflanzlichen Ölen mit einem Gehalt an mindestens einer mehrfach ungesättigten Fettsäure,

Die erfindungsgemäße Zusammensetzung enthält bevorzugt mindestens 1 Gew.-%, bevorzugter mindestens 2,5 Gew.-%, noch bevorzugter mindestens etwa 5 Gew.-% am meisten bevorzugt mindestens etwa 10 Gew.-% eines Fettes und/oder Öls, Insbesondere eines pflanzlichen Öls, mit einem Gehalt an mindestens einer mehrfach ungesättigten Fettsäure.

Der Gehalt an mehrfach ungesättigten Fettsäuren In der Zusammensetzung beträgt bevorzugt mindestens 0,1 Gew.-%, bevorzugter mindestens etwa 1 Gew.-%, besonders bevorzugt mindestens etwa 2,5 Gew.-% bezogen auf die Gesamtmenge der Zusammensetzung. Er kann gaschromatograpisch wie vorstehend erwähnt als Gehalt der extrahierbaren Fettsäuren bestimmt werden, oder er wird näherungsweise aus den gegebenen Fettsäurezusammensetzungen der verwendeten Fette und/oder Öle (P. Schweiger, S. Kerschmann in "Untersuchungen über die Fettsäure- und Tocopherolgehalte von Pflanzenölen", ibid.) bestimmt, wobei näherungsweise der Gewichtsanteil der Fettsäure, bezogen auf die Gesamtmenge der Fettsäuren, dem Gewichtsanteil der Fettsäure in dem Öl gleichgesetzt werden kann.

Die Fettsubstanzen können allein oder in Kombination miteinander verwendet werden. Besonders bevorzugt werden die Fettsubstanzen aus pflanzlichen Ölen ausgewählt. Ganz besonders bevorzugt enthält die erfindungsgemäße Zusammensetzung Sonnenblumenöl mit seinem hohen Gehalt an essentiellen, mehrfach ungesättigten Fettsäuren (P/S-Quotient etwa 5,4). Besonders bevorzugt enthält die erfindungsgemäße Zusammensetzung eine Mehrzahl von pflanzlichen Ölen mit einem Gehalt an mehrfach ungesättigten Fettsäuren. Eine bevorzugte Ausführungsform enthält als Fettsubstanz eine Mischung aus Sonnenblumenöl, Avocadoöl, Nachtkerzenöl und Sesamöl.

In einer bevorzugten Ausführungsform umfassen die Fettsubstanzen mindestens ein γ-Linolensäure-haltiges pflanzliches Öl, dass gegebenenfalls mit anderen nicht γ-Linolensäure-haltigen pflanzlichen Ölen kombiniert werden kann. In einer bevorzugten Ausführungsform enthält die erfindungsgemäße Zusammensetzung bevorzugt mindestens ein γ-Linolensäure-haitiges pflanzliches Öl und mindestens ein nicht γ-Linolensäure-haltlges pflanzliches Öl (< 0,01 Gew.-% bezogen auf die gesamten Fettsäuren des Öls).

Die erfindungsgemäße Zusammensetzung weist einen Gehalt von γ-Linolensäure von mindestens 0,005 Gew.-%, bevorzugt 0,01 Gew.-% bezogen auf das Gewicht der gesamten Zusammensetzung auf. Der Gehalt der γ-Linolensäure in der vorliegenden Erfindung kann, wie zuvor im Zusammenhang mit den mehrfach ungesättigten Fettsäuren beschrieben, bestimmt werden. Ein Gehalt von γ-Linolensäure im Zusammenhang mit der vorliegenden Erfindung bedeutet, dass in der erfindungsgemäßen Zusammensetzung γ-Linolensäure und/oder γ-Linolensäure-Verbindungen, einschließlich ihrer Ester und Salze, vorliegen können. Bevorzugt wird die γ-Linolensäure in der Form ihrer Ester, bevorzugter In der Form ihrer Glycerinester und ganz besonders bevorzugt In der Form γ-Linolensäure-haltiger Pflanzenöle, wie insbesondere dem Samenöl von Nachtkerze (*Oenothera blennis*, γ-Linolensäure-Gehalt ca. 10 %), schwarzer Johannisbeere (*Ribes nigrum*, γ-Linolensäure-Gehait ca. 15 %), Borretsch (*Borago officinalis*, γ-Linolensäure-Gehalt ca. 20-25 %) und anderen Rauhblattgewächsen, sowie Hanföl (Linolensäure-Gehalt ca. 20 %, davon etwa 3 % γ-Linolensäure) der erfindungsgemäßen Zusammensetzung zugesetzt.

Unter den γ-Linolensäure-haltigen Pflanzenölen wird besonders bevorzugt das Nachtkerzenöl verwendet.

Die Bestimmung des γ-Linolensäure-Gehalts der Zusammensetzung kann wie vorstehend bei den mehrfach ungesättigten Fettsäuren erwähnt gaschromatographisch erfolgen. Näherungsweise kann der γ-Linolensäure-Gehalt aber auch aus den Fettsöurezusammensetzungen der bevorzugt verwendeten pflanzlichen Ölen und/oder Fetten und deren verwendeten Mengen bestimmt werden. Der γ-Linolensäure-Gehalt In der erfindungsgemäßen Zusammensetzung beträgt bevorzugter mindestens etwa 0,01 Gew.-%, noch bevorzugter mindestens etwa 0,03 Gew.-% und ganz besonders bevorzugt mindestens etwa 0,05 Gew.-%. Er kann aber auch oberhalb von 0,07 Gew.-% liegen.

Je nach γ-Linolensäure-Gehalt der bevorzugt verwendeten γ-Linolensäure-haltigen pflanzlichen Öle enthält die erfindungsgemäße Zusammensetzung zweckmäßig mindestens etwa 0,1 Gew.-% dieser Öle, wie z.B. Nachtkerzenöl oder Borretschöl, bevorzugt mindestens etwa 0,2 bis 20 Gew.-% γ-Linolensäure-haitige pflanzliche Öle.

Die erfindungsgemäße Zusammensetzung enthält weiterhin Wasser. D.h., es handelt sich bei der erfindungsgemäßen Zusammensetzung um eine Emulsionszusammensetzung. Bei dem eingesetzten Wasser handelt es sich um übliches, für die Herstellung kosmetischer oder pharmazeutischer Formulierungen verwendetes Wasser.

Der Wassergehalt der erfindungsgemäße Zusammensetzung liegt bevorzugt bei etwa 15 bis 95 Gew.-%, bevorzugter bei etwa 30 bis 90 Gew.-%, besonders bevorzugt bei etwa 30 bis 70 Gew.-% am meisten bevorzugt bei etwa 30 bis 60 Gew.-%.

Die erfindungsgemäße Zusammensetzung enthält gegebenenfalls mindestens einen Emulgator. Dabei kann es sich um einen für kosmetische und pharmazeutische Emusionsformulierungen üblichen Emulgator handeln. Bevorzugt werden Emulgatoren auf pflanzlicher Basis verwendet.

Die erfindungsgemäße Zusammensetzung sowie die daraus hergestellten Formulierungen liegen als Wasser-in-Öl-(W/O)-Emulsion oder als Öl-in-Wasser-(O/W)-Emulsion vor. Bevorzugt ist eine Wasser-In-Öl-(W/O)-Emulsion.

Die erfindungsgemäße Zusammensetzung, die, wie oben beschrieben, Glycerin, mindestens eine Fettsubstanz, enthaltend mindestens eine mehrfach ungesättigten Fettsäure in freier oder chemisch gebundener Form, Wasser sowie gegebenenfalls mindestens einen Emulgator enthält, kann als solche bereits als kosmetische Formulierung zur äußerlichen Anwendung verwendet werden, oder sie kann weitere pharmazeutisch oder kosmetisch wirksame Bestandteile enthalten. Als weitere pharmazeutische bzw. kosmetische Wirkstoffe sind Zinkoxid, elementarer Schwefel bzw. Schwefelverbindungen, Vitamine, wie z.B. Vitamin E, Allantoin und pflanzliche Extrakte geeignet. Die weiteren pharmazeutisch oder kosmetisch wirksamen Bestandteile können in reiner oder in gelöster oder dispergierter Form der erfindungsgemäßen Zusammensetzung in einer Menge von beispielsweise etwa 0,01 bis 5 Gew.-% bezogen auf die erfindungsgemäße Zusammensetzung zugemischt werden.

Die erfindungsgemäße Zusammensetzung wird zweckmäßig durch ein Verfahren hergestellt, bei dem die oben genannten Bestandteil zusammengegeben und bei Temperaturen von etwa 60 bis 200 °C emulgiert werden.

Die Erfindung betrifft weiterhin die nicht therapeutische Verwendung der erfindungsgemäßen Zusammensetzung als kosmetische Formulierung nicht-therpeutische zur Behandlung trockener Haut, und/oder zur regenerativen Behandlung der Haut.

Die lediglich aus dem Glycerin, mindestens einer Fettsubstanz, enthaltend mindestens eine mehrfach ungesättigten Fettsäure In freier oder chemisch gebundener Form, Wasser sowie gegebenenfalls mindestens einem Emulgator bestehende Zusammensetzung kann auch als Grundlage für pharmazeutische und/oder kosmetische Formulierungen anderer pharmazeutischer und/oder kosmetischer Wirkstoffe in anderen Indikationsgebieten dienen (nicht beansprucht).

Die Erfindungsgemäßen Zusammensetzungen liegen als Emulsionen, beispielsweise In der Form einer Creme, einer Lotion, einer dickflüssigen Lotion, eines Gel, einer Milch, einer Paste etc. vor. Dem Fachmann ist bekannt, wie er die verschiedenen Emulsionstypen abhängig vom Herstellungsverfahren und der Zusammensetzung herstellen kann. Besonders bevorzugt liegt die erfindungsgemäße Zusammensetzung in der Form einer Creme vor.

Die Zusammensetzung der Erfindung kann bei Bedarf neben den eigentlichen pharmazeutischen und/oder kosmetischen Wirkstoffen gegebenenfalls in solchen Formulierungen übliche Zusätze, ausgewählt aus Verdickungsmittel, Parfums, flächenaktiven Stoffen, Füllstoffen, Mitteln zum Ansäuern oder Alkalischmachen, Färbemitteln, Pigmenten oder Nanopigmenten, wie Titanoxid (amorph oder kristallin in Form von Rutil und/oder Anatas), Oxiden von Eisen, Zirconium oder Cer, die beispielsweise als Lichtschutzmittel bekannt sind, enthalten.

Bevorzugt enthält die Zusammensetzung jedoch nur die oben genannten wesentlichen Bestandteile sowie gegebenenfalls weitere pharmazeutisch oder kosmetisch wirksame Bestandteile.

## Patentansprüche

1. Kosmetische Zusammensetzung zur äußerlichen kosmetischen Anwendung bei Menschen oder Tieren, bestehend aus:
- 12 bis 20 Gew.-% Glycerin, bezogen auf die Gesamtmenge der Zusammensetzung,
- mindestens 20-55 Gew.-%, bezogen auf die Gesamtmenge der Zusammensetzung, einer Fettsubstanz pflanzlicher Herkunft enthaltend mindestens eine mehrfach ungesättigte Fettsäure in freier oder chemisch gebundener Form,
- Wasser,
- gegebenenfalls einen oder mehrere Emulgatoren sowie
- gegebenenfalls einen oder mehrere kosmetische und/oder pharmazeutische Wirkstoffe; ausgewählt aus Allantoin, Vitaminen, Zinkoxid, elementarem Schwefel, Schwefelverbindungen und Pflanzenextrakten,
- sowie gegebenenfalls einen oder mehrere übliche Zusatzstoffe, ausgewählt aus Verdickungsmitteln, grenzflächenaktiven Stoffen, Füllstoffen, Mitteln zum Ansäuern oder Alkalischmachen, Färbemitteln, Pigmenten, Nanopigmenten und Lichtschutzmitteln, Parfum,
wobei die Zusammensetzung einen γ-Linolensäure-Gehalt von mindestens 0,005 Gew.-%, bezogen auf die Gesamtmenge der Zusammensetzung aufweist,
und wobei die Zusammensetzung keine Wachse, keine Paraffine und keine Silikonöle enthält.

2. Zusammensetzung nach Anspruch 1, wobei die Fettsubstanz mindestens ein Fett und/oder Öl mit einem Gehalt an mindestens einer mehrfach ungesättigten Fettsäure umfasst.

3. Zusammensetzung nach einem der Ansprüche 1 bis 2, wobei das Fett und/oder Öl aus pflanzlichen Ölen ausgewählt wird.

4. Zusammensetzung nach Anspruch 1, **dadurch gekennzeichnet, dass** die γ-Linolensäure als Glycerinester enthalten ist.

5. Zusammensetzung nach Anspruch 1, **dadurch gekennzeichnet, dass** die γ-Linolensäure in der Form mindestens eines γ-Linolensäure-haltigen pflanzlichen Öls zugesetzt wird.

6. Zusammensetzung nach Anspruch 5, **dadurch gekennzeichnet, dass** das mindestens eine γ-Linolensäure enthaltende pflanzliche Öl aus der Gruppe ausgewählt wird, die aus dem Samenöl der Nachtkerze (*Oenothera biennis*), dem Samenöl der schwarzen Johannisbeere (*Ribes nigrum*) oder dem Samenöl des Borretsch (*Borago officinalis*) besteht.

7. Zusammensetzung nach einem der Ansprüche 1 bis 6, die als Wasser-in-ÖI-(W/O)-Emulsion vorliegt.

8. Zusammensetzung nach einem der Ansprüche 1 bis 7, enthaltend mindestens einen pharmazeutischen und/oder kosmetischen Wirkstoff, ausgewählt aus Allantoin, Vitaminen, Zinkoxid, elementarem Schwefel, Schwefelverbindungen und Pflanzenextrakten.

9. Verfahren zur Herstellung der Zusammensetzung nach einem Ansprüche 1 bis 8, worin die Bestandteile bei Temperaturen von 60 bis 200 °C emulgiert werden.

10. Nicht-therapeutische Verwendung einer Zusammensetzung nach einem der Ansprüche 1 bis 9 zur Behandlung von trockener Haut und/oder zur regenerierenden Behandlung der Haut.

## Claims

1. A cosmetic composition for external cosmetic use in humans or animals, consisting of:
- 12 to 20 wit.% glycerol, based on the total amount of the composition,
- at least 20 - 55 wit.%, based on the total amount of the composition, of a fatty substance of vegetable origin, containing at least one polyunsaturated fatty acid in free or chemically form,
- water,
- optionally one or more emulsifiers as well as
- optionally one or more active cosmetic and/or pharmaceutical ingredients selected from allantoin, vitamins, zinc oxide, elementary sulfur, sulfur compounds and plant extracts,
- as well as optionally one or more usual additives, selected from thickeners, surface active agents, fillers, agents for acidifying or alkalinizing, dying agents, pigments, nanopigments and sunscreens, perfume,
wherein the composition has a γ-linolenic acid content of at least 0.005 wt.%, based on the total amount of the composition, and
wherein the composition contains no waxes, no paraffins and no silicon oils.

2. The composition according to claim 1, wherein the fatty substance comprises at least one fat and/or oil having a content of at least one polyunsaturated fatty acid,

3. The composition according to one of claims 1 and 2, the fat and/or oil is selected from vegetable oils.

4. The composition according to Claim 1, **characterised in that** the γ-linolenic acid is contained as the glycerol ester,

5. The composition according to Claim 1, **characterised In** the γ-linolenic acid is added in the form of at least one vegetable oil containing γ-linolenic acid.

6. The composition according to Claim 5, **characterised in that** the at least one vegetable oil containing γ-linolenic acid is selected from the group consisting of the seed oil of evening primrose (*Oenothera biennis*), the seed oil of blackcurrant (*Ribes nigrum*) or the seed oil of borage (*Borago officinalis*).

7. The composition according to of Claims 1 to 6, which is present as a water-in-oll (W/O) emulsion.

8. The composition according to one of Claims 1 to 7, containing at least one active pharmaceutical and/or cosmetic ingredient selected from allantoin, vitamins, zinc oxide, elementary sulfur, sulfur compounds and plant extracts.

9. A process for the production of the composition according to one of Claims 1 to 8, wherein the components are emulsified at temperatures of 60 to 200°C,

10. Non-therapeutic use of the composition according to one of Claims 1 to 9 for the treatment of dry skin and/or the regenerative treatment of the skin.

## Revendications

1. Composition cosmétique pour l'application cosmétique extérieure chez l'homme ou chez des animaux consistant en :
- 12 à 20 % en poids de glycérol, par rapport à la quantité totale de la composition,
- au moins 20-55 % en poids par rapport à la quantité totale de la composition d'une substance grasse d'origine végétale, contenant au moins un acide gras polyinsaturé sous forme libre ou chimiquement liée,
- de l'eau,
- éventuellement un ou plusieurs et
- éventuellement un ou plusieurs principes actifs cosmétiques et/ou pharmaceutiques, sélectionnés parmi l'allantoïne, les vitamines, l'oxyde de zinc, le soufre élémentaire, les composés de soufre et les extraits de plantes,
- et éventuellement un ou plusieurs additifs, sélectionnés parmi les épaississants, les tensioactifs, les charges, les agents acidifiants, les agents basifiants, les colorants, les pigments, les nanoparticules, et les stabilisants à la lumière, le parfum,
dans laquelle la composition présente une teneur en acide γ-linoléique d'au moins 0,005 % en poids, par rapport à la quantité totale de la composition, dans laquelle la composition ne contient pas des cires, des paraffines et des huiles de silicones.

2. Composition selon la revendication 1 dans laquelle la substance grasse comprend au moins une graisse et/ou une huile ayant une teneur en au moins un acide gras polyinsaturé.

3. Composition selon l'une quelconque des revendications 1 ou 2 dans laquelle la graisse ou l'huile est sélectionnée parmi les huiles végétales.

4. Composition selon la revendication **caractérisée par le fait que** l'acide γ-linoléique et contenu en tant que l'ester de glycérol.

5. Composition selon la revendication 1 **caractérisée par le fait que** l'acide γ-linoléique est ajouté en forme d'au moins une huile végétale contenant de l'acide γ-linoléique.

6. Composition selon la revendication 5 **caractérisée par le fait que** le au moins une huile végétale contenant de l'acide γ-linoléique est sélectionnée parmi le groupe consistant en l'huile de graines d'onagre (*Oenothera biennis*), l'huile de graines de cassis (*Ribes nigrum* ) ou l'huile de graines de bourrache (Borago officinalis).

7. Composition selon l'une quelconque des revendications 1à 6, se présentant sous la forme d'une émulsion de type eau-dans-l'huile (W/O).

8. Composition selon l'une quelconque des revendications 1à 7, contenant au moins un principe actif cosmétique et/ou pharmaceutique, sélectionné parmi l'allantoïne, les vitamines, l'oxyde de zinc, le soufre élémentaire, les composés de soufre et les extraits de plantes.

9. Procédé de fabrication de la composition selon l'une quelconque des revendications 1 à 1 à 1 8, dans lequel les composants sont émulsionnés des à des températures de 60 à 200 °C.

10. Utilisation non-thérapeutique d'une composition selon l'une quelconque des revendications 1 à 9 pour le le traitement d'une peau sèche et/ou pour le traitement régénérant de la peau.
